# EUROPEAN PATENT SPECIFICATION

(11) **EP 1 011 491 B1**
(45) Date of publication and mention of the grant of the patent: **17.01.2007**
(21) Application number: 98913208.9
(22) Date of filing: 27.03.1998
(51) Int. Cl.: A61B 18/00

(54) **GLASS CORE GUIDEWIRE COMPATIBLE WITH MAGNETIC RESONANCE**
MIT MAGNETRESONANZ KOMPATIBLER GLASKERNFÜHRUNGSDRAHT
FIL DE GUIDAGE A AME EN VERRE COMPATIBLE AVEC LA RESONANCE MAGNETIQUE

(30) Priority: 27.03.1997 NL 1005662
(43) Date of publication of application: 28.06.2000
(73) Proprietor: Cordis Corporation, Miami Lakes, Florida 33014 (US); Hurtak, Wenzel Franz, 9301 TZ Roden (NL); Mous, Frans, 9403 HP Drachten (NL); Nap, Cornelis Philipus, 9345 AG Zevenhuizen (NL)
(72) Inventor: HURTAK, Wenzel, Franz, NL-9301 TZ Roden (NL); MOUS, Frans, NL-9403 HP Drachten (NL); NAP, Cornelis, Philipus, NL-9345 AG Zevenhuizen (NL)
(74) Representative: Mercer, Christopher Paul
(86) International application number: PCT/US1998/006080
(87) International publication number: WO 1998/042268

(56) References cited:
- EP-A- 0 317 091
- EP-A- 0 519 604
- EP-A- 0 597 341
- EP-A- 0 744 186
- WO-A-96/26671
- US-A- 4 832 023
- US-A- 5 217 026
- US-A- 5 251 640
- US-A- 5 439 000
- US-A- 5 601 087
- US-A- 5 690 120

## Description

### BACKGROUND AND SUMMARY OF THE INVENTION

### 1. Technical Background:

The present invention relates generally to intravascular medical devices, and more particularly, to a medical guidewire for use with magnetic resonance systems. Such guidewires may be used in medical procedures for both diagnostic and interventional purposes.

### 2. Discussion:

Guidewires are used in a wide variety of medical procedures, most often in conjunction with one or more other medical devices, including catheters. Such a catheter may be any of various types, such as angiography or angioplasty, but should in any event have a tubular lumen or other guiding means through which the guidewire can be advanced or withdrawn.

Structurally, guidewires are often long, thin metal wires that generally taper from one diameter at a proximal end which remains outside the body of the patient, to a smaller diameter at the opposite distal end. Specifically, vascular guidewires are often more than 1,52 m (five feet) long and have a maximum outer diameter of approximately 0,96 mm (0.038 inches). The diameter of the core wire is generally ground down precisely in a series of alternating tapering portions and constant diameter sections, to develop a selectively engineered flexibility profile along the length of the guidewire.

The guidewire distal tip is usually very flexible, both to avoid vascular trauma and so that it can be selectively bent and twisted to advance it along a desired vascular path. Guidewires are designed to resist this twisting force or torsion, so that as the guidewire proximal end is twisted or rotated, the distal tip tends to rotate through about the same angle. In addition, a floppy spring is often affixed to the extreme distal tip of the guidewire for flexibility.

A good example of a current guidewire is described in United States Patent number 4,846,186, issued to Box et al. on July 11, 1989.

The Box patent shows a guidewire suitable for both diagnostic and therapeutic or interventional procedures, having a Teflon coating from the proximal end along a majority of its length. The core wire tapers in steps to a distal portion that is flattened and surrounded by a flexible spring, which is brazed to the extreme distal end of the core wire to form a rounded tip.

As the body of the patient is of course opaque, physicians commonly use fluoroscopy or X-ray video cameras to track the position of the guidewire and to construct real-time images of the patient's vasculature. The visibility and brightness of selected portions of the guidewire is a relatively important feature, as described in United States Patent number 5,259,393, issued to Corso, Jr. et al. on November 9, 1993, and United States Patent number 5,267,574, issued to Viera et al. on December 7, 1993.

In the Corso patent, the flexible spring at the guidewire distal tip is arranged to selectively control its brightness on an X-ray fluoroscope, or radiopacity. Likewise, the Viera patent discloses a plastic sleeve shrunk around an intermediate section of the guidewire, and several radiopaque marker bands.

In contrast to fluoroscopy, another method of visualizing the patient is magnetic resonance imaging, referred to as MRI. Other medical fields, such as neurology, often use procedures which are performed under MRI instead of X-ray fluoroscopy. Accordingly, it is also desirable to image the anatomy and to track the position of intravascular devices, including catheters and guidewires, using magnetic resonance (MR) systems.

For these applications, it is desirable to make guidewires usable and compatible with MRI techniques. However, a metal guidewire may be too visible under MR, brightly washing out the screen and obscuring important features. This halo phenomenon is called an "artifact," and renders the image useless. Another issue with the use of a metal guidewire under MR is the induction of eddy currents in the metal, caused by distortion of the magnetic field. These eddy currents can generate heat and may increase the local temperature of the surrounding tissue and body fluids, thus possibly damaging the tissue or causing the blood to coagulate.

It is an object of the present invention to provide a guidewire having the desired physical features, including torsion and flexibility, while also avoiding the creation of undesirable artifacts in the MR image or the generation of heat.

In WO 96/26671, there is disclosed a medical guidewire of the type set out in the pre-characterising portion of the accompanying claim 1.

According to the present invention, there is provided a medical guidewire as set forth in the accompanying claim 1.

As such, the present invention provides a guidewire compatible for use with magnetic resonance systems, made from a non-metallic material with a high specific electric impedance. Accordingly, this material will resist any electrical eddy currents in the guidewire from being generated by variations in the high-frequency field. An acceptable class of materials is glass, which are all electrical insulators. A guidewire having a major portion constructed of a glass material should therefore have the advantages of not disturbing the MR field and images, as well as resisting the generation of heat.

These and various other objects, advantages and features of the invention will become apparent from the following description and claims, when considered in conjunction with the appended drawings.

### BRIEF DESCRIPTION OF THE DRAWINGS

Figure 1 is a perspective view of a guidewire for use with magnetic resonance systems, arranged according to an embodiment of the present invention;
Figure 2 is a cross-sectional view of a portion of the guidewire of Figure 1, in a location near that indicated by arrow II;
Figure 3 is a cross-sectional view of a portion of the guidewire of Figure 1, in a location near that indicated by arrow III;
Figure 4 is a perspective view of a guidewire for use with magnetic resonance systems, arranged according to another embodiment of the present invention;
Figures 5-7 are cross-sectional views of a portion of various guidewires arranged according to certain embodiments of the present invention;
Figure 8 is a perspective view of a guidewire for use with magnetic resonance systems, arranged according to another embodiment of the present invention;
Figure 9 is a cross-sectional view of a portion of the guidewire of Figure 8, in a location near that indicated by arrow IX;
Figure 10 is a cross-sectional view of a portion of the guidewire of Figure 8, in a location near that indicated by arrow X; and
Figures 11-13 are side elevation views of distal portions of guidewires arranged according to alternative embodiments of the present invention.

### DETAILED DESCRIPTION OF THE PREFERRED EMBODIMENT

The following description of the preferred embodiments of the present invention is merely illustrative in nature, and as such it does not limit in any way the present invention, its application, or uses. Numerous modifications may be made by those skilled in the art without departing from the true scope of the invention as defined by the accompanying claims.

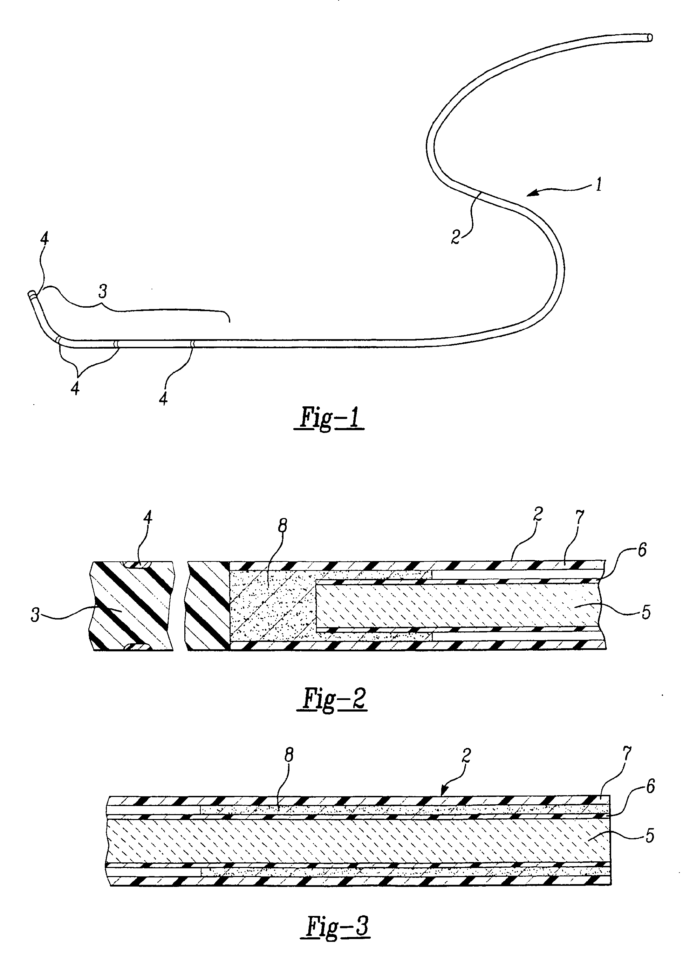

Referring to Figure 1, a perspective view of a guidewire according to a first preferred embodiment of the present invention is shown generally at 1. The medical guidewire 1 is intended for use in intravascular medical procedures involving the use of magnetic resonance systems, including both magnetic resonance imaging and magnetic resonance tracking of the guidewire's position within the body of the patient. Guidewire 1 is constructed of a basic body 2 and a distal tip portion 3. The distal tip of guidewire 1 includes several markers 4 embedded in the distal tip portion 3, which are more visible under MR than the remainder of the guidewire.

The proximal portion of the basic body 2 is illustrated in Figure 3, and incorporates a relatively long, thin core or glass body 5, which may be encased with a protective coating 6 for improving the break strength of the glass body 5. The coated glass body 5 extends for substantially the length of the guidewire and is surrounded with a polymer sheath 7, which is adhered to the glass body 5 with a glue 8.

Markers 4 are visible under MR because their magnetic susceptibility differs to a controlled extent from the remainder of the guidewire and surrounding body tissue, thus distorting the uniformity of the magnetic resonance field, and causing the magnetic field to become what is called "locally inhomogeneous." The material of the markers 4 is selected specifically for this property, and acceptable materials include Dysprosium Oxide (Dy₂O₃).

The glass body 5 is preferably made of a glass material having a high specific electric impedance, such as fiberglass, silica, or quartz.

The coating 6 adds strength to the glass core 5, in that the coating allows the glass core 5 to be bent through a sharper turn or more tortuous path without breaking. Indeed, it has been found that the coated glass core 5 may endure strain as high as 12%. A suitable material for the coating 6 has been found to be polyimide.

The outer polymer sheath 7 may be constructed from any of a variety of materials, including nylon. An additional advantage of the design of the present invention is that the polymer sheath 7 can maintain the physical integrity of the guidewire, even if the glass core 5 should unexpectedly break. Of course, the polymer sheath 7 may be provided with a lubricious or hydrophilic coating, as generally known in the art.

An intermediate portion of the guidewire is depicted in Figure 2, which focuses on a region near the transition at arrow II between the glass core proximal portion of the basic body, referred to as the "transition point."

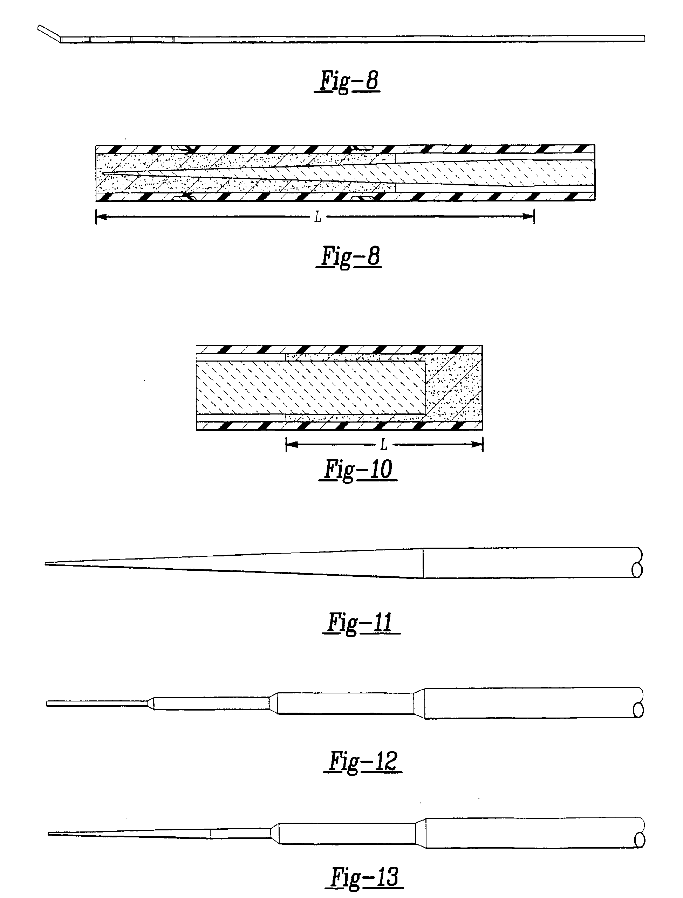

The distal tip portion 3 of the guidewire 1 may be formed of a non-metallic material, such as plastic, as shown in Figure 2, or of a metal as shown in Figures 8-13. The outer diameter of guidewire 1 preferably tapers to a smaller diameter toward the distal tip, as illustrated in Figures 8-13. The metal tip portion may be a material having a selected magnetic susceptibility, such as stainless steel or nickel titanium (nitinol). Preferably, the length of the metal distal tip segment is substantially shorter than the wavelength of the magnetic resonance field in which the guidewire is used.

A short metal collar (not shown) may be affixed to the guidewire at the transition point, to resist kinking and breakage of the guidewire 1 at the transition point.

The glue 8 is preferably of a type that cures upon exposure to ultraviolet light. Accordingly, the polymer sheath 7 should be transparent, to allow the glue 8 to be exposed to the ultraviolet light after portions of the guidewire 1 are assembled as shown in Figures 1-3.

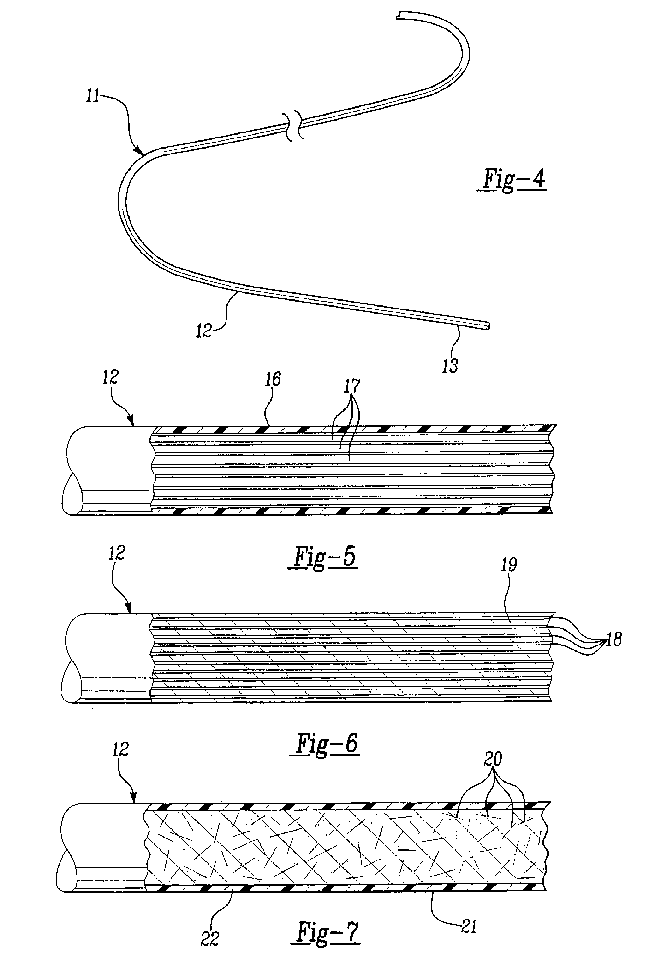

An alternative embodiment of the present invention is depicted in Figures 4-7, in which a guidewire 11 has a proximal portion 12 and a distal tip portion 13. Guidewire 11 has a plastic sheath 16 in which a number of reinforcing fibers have been embedded. Sheath 16 may be shrunk around a bundle of fibers 17, or the sheath 16 may be braided with the reinforcing fibers. Alternatively, fibers 18 may be embedded in a polymer matrix 19. In addition, a multiplicity of short reinforcing fibers 20 can be provided in a polymer matrix 21, surrounded by a coating 12. The reinforcing fibers may be of any suitable material, such as carbon, borium, aramide, or glass.

The guidewire of the present invention may also be constructed of more than one glass core body, all of which may be clad as a unit with a single protective coating.

The distal tip of the guidewire may be bent slightly, to facilitate the selective steering of the guidewire along a desired vascular path.

It should be understood that an unlimited number of configurations for the present invention can be realized. The foregoing discussion describes merely exemplary embodiments illustrating the principles of the present invention, the scope of which is recited in the following claims. Those skilled in the art will readily recognize from the description, claims, and drawings that numerous changes and modifications can be made without departing from the scope of the invention as defined by the accompanying claims.

## Claims

1. A medical guidewire (1;11), for use in intravascular medical procedures and compatible with magnetic resonance, the guidewire having proximal and distal ends and comprising:
a relatively long, thin core (5) extending for substantially the length of the guidewire, the core being made of a glass having a high specific electric impedance; and
a polymer sheath (7,16) surrounding the core (5); **characterised by**
at least one marker (4) positioned near a distal end of the guidewire, wherein the at least one marker (4) is visible under magnetic resonance due to susceptibility-induced magnetic field inhomogeneity, wherein:
either the guidewire (1;11) is formed entirely of non-metallic materials with a high specific electrical impedance,
or the guidewire (1;11) is formed entirely of non-metallic materials with a high specific electrical impedance, except for a relatively short distal tip segment (3;13) made of metal components affixed to the glass core (5) at a transition point, wherein the length of the relatively short distal tip segment (3;13) is shorter than the wavelength of a magnetic resonance field.

2. The medical guidewire of Claim 1, which has said relatively short metal distal tip segment, wherein said tip segment (3;13) is made of nitinol.

3. The medical guidewire of Claim 1 or Claim 2, which has said relatively short metal distal tip segment, wherein the polymer sheath (7;16) extends continuously from a location near the proximal end of the guidewire (1;11) to a location distal of the transition point, thus surrounding at least a portion of both the glass core (5) and the relatively short metal distal tip segment (3;13).

4. The medical guidewire of any one of Claims 1 to 3, which has said relatively short metal distal tip segment (3;13), further comprising a short metal collar affixed to the guidewire (1;11) at the transition point, to resist kinking and breakage of the guidewire (1;11) at the transition point.

5. The medical guidewire of any one of Claims 1 to 4, further comprising a plurality of reinforcing fibers (17;18;20) affixed to the core (5) and/or to the polymer sheath (7;16) to enhance the flexibility and torsion characteristics of the guidewire (1;11).

6. The medical guidewire of Claim 5, wherein the material of the reinforcing fibers (17;18;20) is carbon, borium, aramide or glass.

7. The medical guidewire of any one of Claims 1 to 6, wherein the material of the core (5) is fiberglass, silica or quartz.

8. The medical guidewire of any one of Claims 1 to 7, wherein a distal segment of the glass core (5) tapers to a diameter at the distal end of the guidewire (1;11) that is smaller than the diameter of a major portion of the core.

9. The medical guidewire of any one of Claims 1 to 8, wherein the material of the at least one marker (4) is Dysprosium Oxide (Dy₂O₃).

10. The medical guidewire of any one of Claims 1 to 9, wherein the distal tip (3;13) of the guidewire (1;11) is bent slightly, to facilitate the selective steering of the guidewire (1;11) along a desired vascular path.

11. The medical guidewire of any one of Claims 1 to 10, wherein the core (5) has a breaking strength, said core (5) being covered with a coating (6;12) for increasing said breaking strength.

12. The medical guidewire of any one of Claims 1 to 11, wherein at least a portion of the polymer sheath (7;16) has a hydrophilic coating.

13. The medical guidewire of any one of Claims 1 to 12, wherein the core (5) is formed of a plurality of glass core strands.

## Patentansprüche

1. Medizinischer Führungsdraht (1; 11) zur Verwendung bei intravaskulären medizinischen Prozeduren und kompatibel mit magnetischer Resonanz, wobei der Führungsdraht ein proximales und distales Ende aufweist und umfaßt:
einen relativ langen dünnen Kern (5), der sich über im wesentlichen die Länge des Führungsdrahts erstreckt, wobei der Kern aus einem Glas mit einer hohen spezifischen elektrischen Impedanz hergestellt ist; und
eine Polymerhülle (7; 16), die den Kern (5) umgibt; **gekennzeichnet durch** zumindest eine Markierung (4), die in der Nähe eines distalen Endes des Führungsdrahts angeordnet ist, wobei die zumindest eine Markierung (4) unter magnetischer Resonanz aufgrund einer suszeptibilitätsinduzicrten Magnetfeldinhomogenität sichtbar ist, wobei:
entweder der Führungsdraht (1; 11) vollständig aus nichtmetallischen Materialien mit einer hohen spezifischen elektrischen Impedanz ausgebildet ist;
oder der Führungsdraht (1; 11) vollständig aus nichtmetallischen Materialien mit einer hohen spezifischen elektrischen Impedanz ausgebildet ist, mit Ausnahme eines relativ kurzen distalen Spitzenabschnitts (3; 13), der aus Metallkomponenten hergestellt ist, die am Glaskern (5) an einem Übergangspunkt befestigt ist, wobei die Länge des relativ kurzen distalen Spitzenabschnitts (3; 13) kürzer ist als die Wellenlänge eines magnetischen Resonanzfelds.

2. Medizinischer Führungsdraht nach Anspruch 1, der den relativ kurzen metallischen distalen Spitzenabschnitt aufweist, wobei der Spitzenabschnitt (3; 13) aus Nitinol hergestellt ist.

3. Medizinischer Führungsdraht nach Anspruch 1 oder 2, der den relativ kurzen metallischen distalen Spitzenabschnitt aufweist, wobei die Polymerhülle (7; 16) sich kontinuierlich von einem Ort in der Nähe des proximalen Endes des Führungsdrahts (1; 11) zu einem Ort distal zum Übergangspunkt erstreckt, so daß zumindest ein Teil sowohl des Glaskerns (5) als auch des relativ kurzen metallischen distalen Spitzenabschnitts (3; 13) umgeben ist.

4. Medizinischer Führungsdraht nach einem der Ansprüche I bis 3, der den relativ kurzen metallischen distalen Spitzenabschnitt (3; 13) und des weiteren eine kurze Metallmanschette aufweist, die an dem Führungsdraht (1; 11) am Übergangspunkt befestigt ist, um einem Knicken und Brechen des Führungsdrahts (1; 11) am Übergangspunkt zu widerstehen.

5. Medizinischer Führungsdraht nach einem der Ansprüche 1 bis 4, der des weiteren mehrere Verstärkungsfasern (17; 18 und 20) aufweist, die an dem Kern (5) und/oder der Polymerhülle (7; 16) befestigt sind, um die Flexibilität und Torsionseigenschaften des Führungsdrahts (1; 11) zu erhöhen.

6. Medizinischer Führungsdraht nach Anspruch 5, wobei das Material der Verstärkungsfasem (17; 18; 20) Kohlenstoff, Bor, Aramid oder Glas ist.

7. Medizinischer Führungsdraht nach einem der Ansprüche 1 bis 6, wobei das Material des Kerns (5) Fiberglas, Siliziumdioxid oder Quarz ist.

8. Medizinischer Führungsdraht nach einem der Ansprüche 1 bis 7, wobei sich ein distaler Abschnitt des Glaskerns (5) am distalen Ende des Führungsdrahts (1; 11) auf einen Durchmesser verjüngt, der kleiner ist als der Durchmesser eines Hauptabschnitts des Kerns.

9. Medizinischer Führungsdraht nach einem der Ansprüche 1 bis 8, wobei das Material der zumindest einen Markierung (4) Dysprosiumoxid (Dy₂O₃) ist.

10. Medizinischer Führungsdraht nach einem der Ansprüche 1 bis 9, wobei die distale Spitze (3; 13) des Führungsdrahts (1; 11) leicht gebogen ist, um das selektive Steuern des Führungsdrahts (1; 11) entlang eines gewünschten vaskulären Wegs zu erleichtern.

11. Medizinischer Führungsdraht nach einem der Ansprüche 1 bis 10, wobei der Kern (5) eine Bruchstärke aufweist, der Kern (5) mit einer Beschichtung (6; 12) überzogen ist, um die Bruchstärke zu erhöhen.

12. Medizinischer Führungsdraht nach einem der Ansprüche 1 bis 11, wobei zumindest ein Teil der Polymerhülle (7; 16) eine hydrophile Beschichtung umfaßt.

13. Medizinischer Führungsdraht nach einem der Ansprüche 1 bis 12, wobei der Kern (5) aus mehreren Glaskernadern ausgebildet ist.

## Revendications

1. Fil de guidage médical (1 ; 11), destiné à être utilisé dans des procédés médicaux intravasculaires et compatible avec la résonance magnétique, le fil de guidage possédant des extrémités distales et proximales, et comprenant :
• une âme mince et relativement longue (5) s'étendant sensiblement sur la longueur du fil de guidage, l'âme étant réalisée dans du verre présentant une impédance électrique spécifique élevée ; et
• une gaine polymère (7 ; 16) entourant l'âme (5) ; **caractérisé par**
• au moins un marqueur (4) positionné à proximité d'une extrémité distale du fil de guidage, dans lequel l'au moins un marqueur (4) est visible lors d'une résonance magnétique en raison de l'inhomogénéité du champ magnétique induit par susceptibilité, dans lequel :
• le fil de guidage (1 ; 11) est formé entièrement dans des matériaux non métalliques avec une impédance électrique spécifique élevée ;
• ou le fil de guidage (1 ; 11) est formé entièrement dans des matériaux non métalliques avec une impédance électrique spécifique élevée, à l'exception d'un segment de bout distal relativement court (3 ; 13) réalisé dans des composants métalliques fixés sur l'âme de verre (5) au niveau d'un point de transition, dans lequel la longueur du segment de bout distal relativement court (3 ; 13) est plus courte que la longueur d'onde d'un champ de résonance magnétique.

2. Fil de guidage médical selon la revendication 1, qui possède ledit segment de bout distal métallique relativement court, dans lequel ledit segment de bout (3 ; 13) est réalisé en nitinol.

3. Fil de guidage médical selon l'une quelconque des revendications 1 ou 2, qui possède ledit segment de bout distal métallique relativement court, dans lequel la gaine polymère (7 ; 16) s'étend de manière continue à partir d'un endroit situé à proximité de l'extrémité proximale du fil de guidage (1 ; 11) jusqu'à un endroit distal du point de transition, en entourant de ce fait au moins une partie de l'âme de verre (5) et le segment de bout distal métallique relativement court (3 ; 13).

4. Fil de guidage médical selon l'une quelconque des revendications 1 à 3, qui possède ledit segment de bout distal métallique relativement court (3 ; 13), comprenant de plus un collier métallique court fixé au fil de guidage (1; 11) au niveau du point de transition, pour résister à un vrillage et à une rupture du fil de guidage (1 ; 11) au niveau du point de transition.

5. Fil de guidage médical selon l'une quelconque des revendications 1 à 4, comprenant de plus une pluralité de fibres de renforcement (17 ; 18 ; 20) fixées sur l'âme (5) et / ou la gaine polymère (7 ; 16), pour améliorer les caractéristiques de souplesse et de torsion du fil de guidage (1 ; 11).

6. Fil de guidage médical selon la revendication 5, dans lequel le matériau des fibres de renforcement (17 ; 18 ; 20) est le carbone, le bore, l'aramide ou le verre.

7. Fil de guidage médical selon l'une quelconque des revendications 1 à 6, dans lequel le matériau de l'âme (5) est une fibre de verre, de la silice ou du quartz.

8. Fil de guidage médical selon l'une quelconque des revendications 1 à 7, dans lequel un segment distal de l'âme de verre (5) s'amincit jusqu'à un diamètre, au niveau de l'extrémité distale du fil de guidage (1 ; 11), qui est plus petit que le diamètre d'une partie principale de l'âme.

9. Fil de guidage médical selon l'une quelconque des revendications 1 à 8, dans lequel le matériau de l'au moins un marqueur (4) est l'oxyde de dysprosium (Dy₂O₃).

10. Fil de guidage médical selon l'une quelconque des revendications 1 à 9, dans lequel le bout distal (3 ; 13) du fil de guidage (1 ; 11) est légèrement plié, pour faciliter le guidage sélectif du fil de guidage (1 ; 11) le long d'un trajet vasculaire désiré.

11. Fil de guidage médical selon l'une quelconque des revendications 1 à 10, dans lequel l'âme (5) présente une résistance à la rupture, ladite âme (5) étant recouverte d'un revêtement (6 ; 12) pour accroître ladite résistance à la rupture.

12. Fil de guidage médical selon l'une quelconque des revendications 1 à 11, dans lequel une partie au moins de la gaine polymère (7 ; 16) possède un revêtement hydrophile.

13. Fil de guidage médical selon l'une quelconque des revendications 1 à 12, dans lequel l'âme (5) est constituée d'une pluralité de brins d'âme de verre.
